(19) **Europäisches Patentamt**

**European Patent Office**

**Office européen des brevets**

(11) **EP 1 165 822 B1**

(12) **EUROPEAN PATENT SPECIFICATION**

(45) Date of publication and mention
of the grant of the patent:
**06.10.2004 Bulletin 2004/41**

(51) Int Cl.⁷: **C12P 13/02**, C12P 1/00

(21) Application number: **00917476.4**

(86) International application number:
**PCT/NL2000/000208**

(22) Date of filing: **29.03.2000**

(87) International publication number:
**WO 2000/058490 (05.10.2000 Gazette 2000/40)**

(54) **PRIMARY AMIDE SYNTHESIS FROM CARBOXYLIC ACIDS WITH A LIPASE**

HERSTELLUNG VON PRIMÄREN AMIDEN AUS CARBONSÄUREN DURCH EINE LIPASE

SYNTHESE D'AMIDES PRIMAIRES D'ACIDES CARBOXYLIQUES AVEC UNE LIPASE

(84) Designated Contracting States:
**AT BE CH CY DE DK ES FI FR GB GR IE IT LI LU MC NL PT SE**

(30) Priority: **29.03.1999 NL 1011687**

(43) Date of publication of application:
**02.01.2002 Bulletin 2002/01**

(73) Proprietor: **TECHNISCHE UNIVERSITEIT DELFT 2628 BL Delft (NL)**

(72) Inventors:
- **LITJENS, Michael, Johannes, Jacobus NL-3522 Utrecht (NL)**
- **STRAATHOF, Adrianus, Johannes, Jozef NL-2611 MZ Delft (NL)**
- **JONGEJAN, Jacob, Arie NL-2641 CJ Pijnacker (NL)**
- **HEIJNEN, Josef, Johannes NL-5122 KK Rijen (NL)**

(74) Representative: **Kupecz, A., Drs. c.s. et al Octrooibureau Los en Stigter B.V. Weteringschans 96 1017 XS Amsterdam (NL)**

(56) References cited:
**WO-A-95/07359          WO-A-98/47860**

- **CEROVSKY, V. ET AL.: "C-terminal peptide amidation catalyzed by orange flavedo peptide amidase." ANGEWANDTE CHEMIE. INTERNATIONAL EDITION., vol. 37, no. 13/14, 1998, pages 1885-7, XP002144585 cited in the application**

- **DE ZOETE, M.C. ET AL.: "A new enzymatic one-pot procedure for the synthesis of carboxylic amides from carboxylic acids." JOURNAL OF MOLECULAR CATALYSIS - B: ENZYMATIC, vol. 2, no. 1, 30 September 1996 (1996-09-30), pages 19-25, XP000929871**

- **WOLFF, A. ET AL.: "Potential of enzymatic kinetic resolution using solid substrates suspension: improved yield, productivity, substarte concentration, and recovery." BIOTECHNOLOGY PROGRESS, vol. 15, no. 2, 1 March 1999 (1999-03-01), pages 216-27, XP000929866**

- **LITJENS, M.J.J. ET AL.: "Synthesis of primary amides by lipase-catalysed amidation of carboxylic acids with ammonium salts in an organic solvent." CHEMICAL COMMUNICTAIONS, 7 July 1999 (1999-07-07), pages 1255-6, XP002144586**

- **LITJENS M J J ET AL: "Exploration of Lipase-catalyzed Direct Amidation of Free Carboxylic Acids with Ammonia in Organic Solvents" TETRAHEDRON,NL,ELSEVIER SCIENCE PUBLISHERS, AMSTERDAM, vol. 55, no. 42, 15 October 1999 (1999-10-15), pages 12411-12418, XP004178966 ISSN: 0040-4020**

Remarks:
The file contains technical information submitted after the application was filed and not included in this specification

**Description**

The present invention relates to a method of preparing a reaction product by means of an enzymatically catalyzed reaction of a first reagent A and a second reagent B in a solvent, the first reagent A and the second reagent B each being soluble in the solvent, and the first. reagent A and the second reagent B are capable of forming a salt AB that precipitates in the solvent.

Such a method is impeded by the fact that the precipitated substrates A and B are not available to the enzyme that catalyzes the reaction between A and B. The commonly used practice in the organic chemistry of raising the temperature of the solvent in order to aid the solubility of the reagents A, B is, in connection with the thermic instability of the enzyme, often only possible to a limited extent. In those cases other measures are necessary. For example Cerovský V. et al. (Angew. Chem. Int. Ed. 37 (13/14) 1885-1887) disclose the addition of water to acetonitrile to improve the solubility. The presence of water is from a chemical point of view not favorable for the desire enzymatic amidation of peptides with ammonia. Poor yields are reported obtained in mixtures that lead to the formation of insoluble salts.

The object of the present invention is to provide a method of the kind mentioned in the preamble, which accelerates the conversion of A and B.

To this end the method according to the invention is characterized in that the concentrations of the first reagent A and the second reagent B are chosen such that they comply with the formula (I)

$$[A]_T = [B]_T + (F\text{-}1) \cdot \sqrt{\frac{K}{F}}$$

wherein

$[A]_T$ represents the concentration of the first reagent A in relation to the total volume taken up by the reagents A, B, and the solvent;
$[B]_T$ represents the concentration of the second reagent B in relation to the total volume taken up by the a reagents A, B, and the solvent;
K is the solubility product for the salt AB formed between the first reagent A and the second reagent B,
and F is a factor between 1/10 and 10.

In chemistry, reagents are basically brought together in stoichiometric amounts. There may be reasons for departing from this principle, for instance to suppress side reactions. Also, if the yield of reactions does not approximate 100%, considerations regarding costs may result in an excess of the cheapest reagents being added. In practice however, when stoichiometric amounts are indeed involved, there is always a deviation from the optimal stoichiometric proportions. Applicant has surprisingly found that minor deviations in the ratio between the reagents A and B have considerable consequences for the rate at which the reagents A and B are converted by the enzyme. Selecting the concentrations. for A and B such that they are within the range defined in accordance with the invention by the factor F, proved to produce good yields more quickly.

The term concentration in the present application refers to the molar concentration. Furthermore, reagents are only included in the formula if they are available for precipitation to salt AB or if they are present as salt AB. The invention is in particular applicable to those reactions in which the salt AB has a solubility product of, for example, less than $10^{-3}$ $M^2$, such as less than $3*10^{-4}$ $M^2$ and in particular less than $10^{-4}$ $M^2$ or $3*10^{-5}$ $M^2$.

To avoid the problem of reagents precipitating as salt, the prior art preparation of primary amides using an enzymatically catalyzed reaction involves esterification of the carboxylic acid prior to contacting the ester with the ammonium compound.

De Zoete et al. (Biocatalysis, 10, pp. 307 - 316, 1994) posit that direct ammoniolysis of octanoic acid does not to produce any octanoic amide because of the formation of salts. For this reason they carry out a two-step reaction via the ethylester of octanoic acid.

Öhrner et al. (Enzyme & Microbial Technology 19, pp. 328 - 331, 1996) state that octanoic acid might lead to the undesirable formation of salts, which is the reason why they, too, use ethyl octanoate.

PCT/EP98/01781 discloses a process for making carboxylic amides involving the combination of near-stoichiometric amounts of carboxylic acid and amine at elevated pressure and temperature in the range of 130°C - 150°C. These reactions at high temperature, which favour solubility, are performed in the absence of a catalytic enzyme.

Montet D. et al. describe in Fat Sci. Technol. 91(1) (1989) the synthesis of N-lauryloleylamide using lipase. Montet et al use the substrates in an equimolar ratio. Of several solvents tried, hexane is used because it is a solvent for both substrates.

The formation of primary amides is an important application of the method.

According to a favourable embodiment, F lies between 1/5 and 5, preferably between 1/3 and 3, and more preferably between 1/2 and 2.

Choosing such restricted ranges results in even faster conversion rates. However, it should be noted that with respect to the concentrations of the two reagents, within the ranges mentioned it may be the reaction-kinetic aspects that cause the deviation from the optimal value of 1 for F, as will be obvious to the person skilled in the art.

According to a very favourable embodiment the first reagent A and the second reagent B are added as their salt. This is a simple and effective way of achieving a value of 1 for F.

An important application of the method according to the invention relates to the preparation of a reaction product wherein the first reagent A is an acidic reagent and the second reagent B is a basic reagent. These will readily precipitate in an organic solvent. If the reaction is a condensation reaction, the solvent will preferably be substantially free of the compound that is released during the condensation reaction as simple molecule, such as water, and which might function as solvent (or as medium aiding dissolution).

According to a preferred embodiment, the first acidic reagent A is a carboxylic acid and the second basic reagent B is a compound selected from the group comprising i) ammonia; and ii) a primary amine.

According to an interesting embodiment, the ammonia is added in the form of ammonium bicarbonate, ammonium carbamate, urea, or ammonia-loaded ion exchange resin.

In this way the ammonia becomes available for reaction more gradually. An interesting aspect of the use of urea as source for ammonia, for example by enzymatic hydrolysis, is that water released during the condensation reaction is removed, which promotes the formation of the amide. For every eliminated water molecule two ammonia molecules are released, which means that there is a certain degree of control regarding the release of ammonia. If desired, this or another embodiment described in the present application, may be provided with an ammonia sensor or may employ a chromatographic technique for measuring the concentration of free ammonia.

As enzyme, an enzyme is used selected from lipases, esterases, and peptidases, such as preferably the lipase Candida antarctica lipase B.

This is an efficient manner of forming a primary amide.

The invention will now be elucidated with reference to the following exemplary embodiment.

EXAMPLE 1

A solution of 73 mg of butyric acid dried over 3 Angstrom molecular sieves in 10 ml of methylisobutyl ketone dried over 3 Angstrom molecular sieves, is stirred at 35°C with 80 mg ammonium carbamate (as ammonia source) and 15 mg Candida antarctica lipase B (Novozym 435, activity 11000 PLU/g, Novo Nordisk, Bagsvaerd, Denmark). The solution also comprises 10 µl dodecane per ml solution as internal standard for analysis. Samples were taken at various intervals and immediately after centrifugation (14,300 rpm for 1 min.) analyzed with the aid of gas chromatography. After three days, the initial butyric acid concentration of 83 mmol/l is reduced to 7 mM, and 75 mM of butyramide is formed.

EXAMPLE 2

Four ml of a solution of 395 mg of butyric acid and 120 µl of dodecane (as internal standard) in methylisobutyl ketone dried over 3 Ångstrom molecular sieves is mixed with dried methylisobutyl ketone saturated with ammonia (230 mM) in an amount indicated in the table below and made up to 30 ml with dried methylisobutyl ketone. Thus this solution contains 150 mM of butyric acid, and a concentration of ammonia as indicated in the table. The suspension is stirred at 25°C with 45 mg of *Candida antarctica* lipase B (Novozym 435, activity 11000 PLU/g, Novo Nordisk, Bagsvaerd, Denmark). Under these conditions $K = 1.3 \cdot 10^{-4}$ $M^2$.

| Experimen **t** | NH$_3$ -satur. MIBK[*] (ml) | MIBK[*] (ml) | [NH$_3$] (mM) | F |
|---|---|---|---|---|
| 1 | 20 | 6 | 153 | 0,8 |
| 2 | 22 | 4 | 169 | 0,2 |
| 3 | 24 | 2 | 184 | 0,09 |
| 4 | 26 | 0 | 199 | 0,05 |

[*] dried methylisobutyl ketone

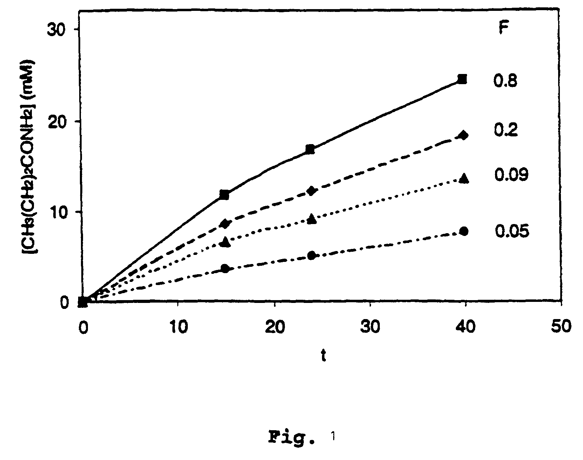

The experiments 3 and 4 are comparative experiments. In the figure the concentration product (butyramide) is plotted against time (in hours). It can be clearly seen that a deviation from the stoichiometric ratio strongly affects the rate at which the product is formed. In experiment 3, the concentration butyric acid is 150 mM and the concentration $NH_3$ is 184 mM. Although the concentration ammonia is only 21% higher, the conversion rate is more than a factor 2 lower than in experiment 1 (F is 0.09 and 0.8, respectively).

EXAMPLE 3

**Preparation of acetamide, the reagents being added as a salt:**

In a 33 ml closed glass vessel 1.25 mmol ammonium acetate (98% pure from J.T. Baker) was stirred in 25 ml dry methylisobutyl ketone containing 10 µl/ml dodecane as an internal standard ($F_{value} \approx 1$). The reaction was started by the addition of 25 mg immobilized Candida antarctica Lipase B (Novozym 435) with a catalytic activity of 11 PLU/mg preparation (which was a kind gift of NOVO Nordisk. Presently this enzyme is marketed by Roche Diagnostics GmbH, Mannheim, Germany). The reaction mixture was stirred at 35°C for 3 days. After that a sample was taken through a septum, centrifuged, and analyzed by GC. The yield was 98%, as determined from the measured acetamide concentration using dodecane as internal standard. GC procedure: acetamide (standard from J.T. Baker) was analyzed on a Shimadzu GC-17A gas chromatograph with a flame ionization detector and a Shimadzu AOC-17 Auto Injector (0.5 µl injection volume). Helium was used as the carrier gas at a split ratio of 1:100. The column used was a Hewlett Packard FFAP Cross-linked Polyethylene Glycol-TPA column (25 m x 0.32 mm) with a retention gap. For analysis of acetic acid and acetamide the column temperature was 80°C for 6 min. and then raised by 15°C.min$^{-1}$ to 110°C. The column pressure was 1.2 bar.

**Claims**

1. A method of preparing a reaction product by means of an enzymatically catalyzed reaction of a first reagent A and a second reagent B in a solvent, the first reagent A and the second reagent B each being soluble in the solvent, and the first reagent A and the second reagent B capable of forming a salt AB that precipitates in the solvent, **characterized in that** the method is performed in the presence of salt AB, where the concentrations of the first reagent A and the second reagent B are chosen such that they comply with the formula (I)

$$[A]_T = [B]_T + (F-1) \cdot \sqrt{\frac{K}{F}}$$

wherein

$[A]_T$ represents the concentration of the first reagent A in relation to the total volume taken up by the reagents A, B, and the solvent;

$[B]_T$ represents the concentration of the second reagent B in relation to the total volume taken up by the a reagents A, B, and the solvent;

K is the solubility product for the salt AB formed between the first reagent A and the second reagent B, and F is a factor between 1/10 and 10.

2. A method according to claim 1, **characterized in that** F lies between 1/5 and 5, preferably between 1/3 and 3, and more preferably between 1/2 and 2.

3. A method according to claim 2, **characterized in that** the first reagent A and the second reagent B are added as salt AB.

4. A method according to one of the preceding claims, **characterized in that** the first reagent A is an acidic reagent and the second reagent B is a basic reagent and the solvent an organic solvent.

5. A method according to claim 4, **characterized in that** the first acidic reagent A is a carboxylic acid and the second basic reagent B is a compound selected from the group comprising i) ammonia; and ii) a primary amine.

6. A method according to claim 4 or 5, **characterized in that** the ammonia is added in the form of ammonium bicarbonate, ammonium carbamate, urea, or ammonia-charged ion exchange resin.

**7.** A method according to one of the claims 4 to 6, **characterized in that** as enzyme for the enzymatically catalyzed reaction an enzyme is used selected from lipases, esterases, and peptidases.

**8.** A method according to claim 7, **characterized in that** the lipase is *Candida antarctica* lipase B.

## Patentansprüche

**1.** Verfahren zur Herstellung eines Reaktionsprodukts mittels einer enzymatisch katalysierten Reaktion eines ersten Reagenzes A und eines zweiten Reagenzes B in einem Lösungsmittel, wobei das erste Reagenz A und das zweite Reagenz B beide in dem Lösungsmittel löslich sind, und wobei das erste Reagenz A und das zweite Reagenz B zur Bildung eines Salzes AB imstande sind, welches in dem Lösungsmittel präzipitiert, **dadurch gekennzeichnet, dass** das Verfahren in der Gegenwart des Salzes AB durchgeführt wird, wobei die Konzentrationen des ersten Reagenzes A und des zweiten Reagenzes B derart ausgewählt werden, dass sie der Formel (I) entsprechen:

$$[A]_T = [B]_T + (F - 1) \cdot \sqrt{\frac{K}{F}}$$

wobei:

$[A]_T$ die Konzentration des ersten Reagenzes A bezogen auf das Gesamtvolumen darstellt, welches von den Reagenzien A, B und dem Lösungsmittel eingenommen wird;
$[B]_T$ die Konzentration des zweiten Reagenz B bezogen auf das Gesamtvolumen darstellt, welches von den Reagenzien A, B und dem Lösungsmittel eingenommen wird;
K das Löslichkeitsprodukt des Salzes AB ist, welches aus dem ersten Reagenz A und dem zweiten Reagenz B gebildet wird;
und F ein Faktor zwischen 1/10 und 10 ist.

**2.** Verfahren gemäß Anspruch 1, **dadurch gekennzeichnet, dass** F zwischen 1/5 und 5, vorzugsweise zwischen 1/3 und 3, und nach bevorzugter zwischen 1/2 und 2 liegt.

**3.** Verfahren gemäß Anspruch 2, **dadurch gekennzeichnet, dass** das erste Reagenz A und das zweite Reagenz B als Salz AB zugegeben werden.

**4.** Verfahren gemäß einem der vorangehenden Ansprüche, **dadurch gekennzeichnet, dass** das erste Reagenz A ein saures Reagenz und das zweite Reagenz B ein basisches Reagenz ist und das Lösungsmittel ein organisches Lösungsmittel.

**5.** Verfahren gemäß Anspruch 4, **dadurch gekennzeichnet, dass** das erste saure Reagenz A eine Carbonsäure und das zweite basische Reagenz B eine Verbindung ist, die aus der Gruppe ausgewählt wird, die umfasst: i) Ammonium, und ii) ein primäres Amin.

**6.** Verfahren gemäß Anspruch 4 oder 5, **dadurch gekennzeichnet, dass** das Ammonium in Form von Ammoniumbicarbonat, Ammoniumcarbamat, Harnstoff oder einem ammonium-beladenen Ionenaustauscherharz zugegeben wird.

**7.** Verfahren gemäß einem der Ansprüche 4 bis 6, **dadurch gekennzeichnet, dass** als Enzym für die enzymatisch katalysierte Reaktion ein Enzym verwendet wird, das aus Lipasen, Esterasen und Peptidasen ausgewählt wird.

**8.** Verfahren gemäß Anspruch 7, **dadurch gekennzeichnet, dass** die Lipase die Lipase B aus *Candida antarctica* ist.

## Revendications

**1.** Procédé pour la préparation d'un produit de réaction au moyen d'une réaction, catalysée enzymatiquement, d'un premier réactif A et d'un second réactif B dans un solvant, le premier réactif A et le second réactif B étant chacun solubles dans le solvant, et le premier réactif A et le second réactif B étant capables de former un sel AB qui précipite dans le solvant, ledit procédé étant **caractérisé en ce qu'**il est mis en oeuvre en présence du sel AB, les concentrations du premier réactif A et du second réactif B- étant choisies de telle sorte qu'elles répondent à la formule (I)

$$[A]_T = [B]_T + (F - 1) \cdot \sqrt{\frac{K}{F}}$$

dans laquelle

[A]$_T$ représente la concentration du premier réactif A par rapport au volume total pris par les réactifs A et B et le solvant ;

[B]$_T$ représente la concentration du second réactif B par rapport au volume total pris par les réactifs A et B et le solvant ;

K représente le produit de solubilité pour le sel AB formé entre le premier réactif A et le second réactif B, et F est un facteur entre 1/10 et 10.

2. Procédé suivant la revendication 1, **caractérisé en ce que** F est compris dans l'intervalle de 1/5 à 5, avantageusement de 1/3 à 3 et plus avantageusement de 1/2 à 2.

3. Procédé suivant la revendication 2, **caractérisé en ce que** le premier réactif A et le second réactif B sont ajoutés sous forme de sel AB.

4. Procédé suivant l'une des revendications précédentes, **caractérisé en ce que** le premier réactif A est un réactif acide et le second réactif B est un réactif basique et le solvant est un solvant organique.

5. Procédé suivant la revendication 4, **caractérisé en ce que** le premier réactif acide A est un acide carboxylique et le second réactif basique B est un composé choisi dans le groupe comprenant i) l'ammoniac et ii) une amine primaire.

6. Procédé suivant la revendication 4 ou 5, **caractérisé en ce que** l'ammoniac est ajouté sous forme de bicarbonate d'ammonium, de carbamate d'ammonium, d'urée ou d'une résine échangeuse d'ions chargée d'ammoniac.

7. Procédé suivant l'une des revendications 4 à 6, **caractérisé en ce que**, comme enzyme pour la réaction catalysée enzymatiquement, on utilise une enzyme choisie entre des lipases, des estérases et des peptidases.

8. Procédé suivant la revendication 7, **caractérisé en ce que** la lipase est la lipase B de *Candida antarctica.*

Fig. 1